(19)

Europäisches
Patentamt
European
Patent Office
Office européen
des brevets

(11)     **EP 3 326 688 B1**

(12)                    **EUROPEAN PATENT SPECIFICATION**

(45) Date of publication and mention
of the grant of the patent:
**07.06.2023   Bulletin 2023/23**

(51) International Patent Classification (IPC):
**A61N 1/36** *(2006.01)*     **A61B 5/00** *(2006.01)*
**A61B 5/369** *(2021.01)*     **A61N 2/00** *(2006.01)*
**A61N 1/04** *(2006.01)*     **A61N 1/05** *(2006.01)*

(21) Application number: **17202536.3**

(22) Date of filing: **08.07.2009**

(52) Cooperative Patent Classification (CPC):
**A61N 1/0476; A61B 5/369; A61B 5/6804;**
**A61B 5/6814; A61N 1/0408; A61N 1/36025;**
A61B 2560/0431; A61N 1/0492; A61N 1/0504;
A61N 1/36017; A61N 1/36046; A61N 2/006

(54) **ELECTRICAL STIMULATION APPARATUS FOR TREATING THE HUMAN BODY**

ELEKTRISCHE STIMULATIONSVORRICHTUNG ZUR BEHANDLUNG DES MENSCHLICHEN
KÖRPERS

APPAREIL DE STIMULATION ÉLECTRIQUE POUR TRAITER LE CORPS HUMAIN

(84) Designated Contracting States:
**AT BE BG CH CY CZ DE DK EE ES FI FR GB GR**
**HR HU IE IS IT LI LT LU LV MC MK MT NL NO PL**
**PT RO SE SI SK SM TR**

(43) Date of publication of application:
**30.05.2018   Bulletin 2018/22**

(62) Document number(s) of the earlier application(s) in
accordance with Art. 76 EPC:
**09164908.7 / 2 272 561**

(73) Proprietor: **Neuromodtronic GmbH**
**14473 Potsdam (DE)**

(72) Inventors:
• **HERWIG, Bernd**
**14055 Berlin (DE)**

• **SABEL, Kornelia**
**14163 Berlin (DE)**
• **FEDOROV, Anton**
**10409 Berlin (DE)**

(74) Representative: **Meissner Bolte Partnerschaft**
**mbB**
**Patentanwälte Rechtsanwälte**
**Postfach 86 06 24**
**81633 München (DE)**

(56) References cited:
**EP-A- 1 145 735     WO-A-2006/089181**
**WO-A-2007/127444     CN-A- 1 089 513**
**US-A1- 2007 123 758     US-A1- 2008 319 511**
**US-B1- 6 934 580**

**Description**

The invention relates to an apparatus for stimulating a patient with an electrical stimulation signal and in particular to an apparatus for treatment of one or several disorders caused by a brain damage or brain disease or disorders of the retina. Human brains are extremely complex. A human brain can contain over 100 billion nerve cells which communicate with one another by means of axons, which carry trains of signal pulses called action potentials. Although the brain is protected by the skull and surrounded by a cerebrospinal fluid and further isolated from the bloodstream by a blood-brain barrier, the delicate nature of the brain makes it vulnerable to numerous diseases and different types of brain damages in which many cells can be lost. These brain disorders can comprise cognitive impairments of the affected patient. Further possible disorders caused by brain damages or brain diseases are movement disorders, memory disorders or other functional neurological disorders. Furthermore, any of the sensory systems (e.g. vision, hearing) can be affected by such brain diseases or - in case of vision - retinal diseases. For example auditory disorders or visual disorders can be caused.

The closest prior art EP 1 145 735 A2 describes a neurostimulator central unit which contains an electrical stimulation source and preferably devices for detecting epileptiform activity. The neurostimulator assembly does also include at least a first brain electrical activity sensor and a responsive electrical neurostimulator electrode. The measuring electrodes are in contact with the brain or scalp electrodes may be located within the brain tissue. Multiple electrodes enhance the ability of electrical stimulation to desynchronize brain activity to terminate epileptiform activity. The device uses various parameters to optimize stimulation to desynchronize the brain activity to terminate epileptiform activity. EP 1 145 735 A2 describes a method where stimulation electrodes are in direct contact with the brain tissue.

Accordingly it is an object of the present invention to provide an apparatus for a treatment of a patient who suffers under a disorder caused by a brain or retinal disease.

This object is achieved by an apparatus comprising the features of claim 1. Preferred embodiments are defined by the dependent claims.

In the following embodiments of the apparatus and the method for stimulating a patient according to the present invention are described with reference to the enclosed figures.

Fig. 1 shows a possible embodiment of a stimulation apparatus according to the present invention attached by means of electrodes to a head of a patient;

Fig. 2 shows a block diagram of a possible embodiment of the apparatus according to the present invention as shown in Fig. 1;

Fig. 3 shows a further block diagram of a possible embodiment of an apparatus according to the present invention as shown in Fig. 1 or 2;

Fig. 4 shows a diagram of a dual electrode which can be used in a possible embodiment of the apparatus according to the present invention;

Fig. 5 shows the connection of a dual electrode as shown in Fig. 4 to circuits within a stimulation apparatus according to a possible embodiment;

Fig. 6 shows a possible embodiment of a dual electrode as used in the stimulation apparatus according to the present invention;

Fig. 7 shows signal diagrams of a stimulation signal and a corresponding EEG-signal used in an exemplary embodiment of the apparatus according to the present invention;

Fig. 8 shows a flow chart of a first embodiment of a method for stimulating a patient as employed by the apparatus according to the present invention;

Fig. 9 shows a flow chart of as second embodiment of a method for stimulation a patient as employed by the apparatus according to the present invention;

Fig. 10a, 10b, 10c show signal diagrams for illustrating a neurophysiological fundamental frequency as employed the apparatus according to the present invention;

Fig. 11 shows a diagram for illustrating the functionality of an apparatus for stimulating a patient according to the present invention;

Fig. 12 shows a signal diagram of an exemplary EEG-signal recorded by an apparatus according to the present invention;

Fig. 13 shows a digram of a frequency spectrum calculated from the EEG-signal shown in Fig. 12 by the apparatus according to the present invention;

Fig. 14 shows further exemplary signal diagrams for illustrating a further possible embodiment of the apparatus for stimulation a patient according to the present invention.

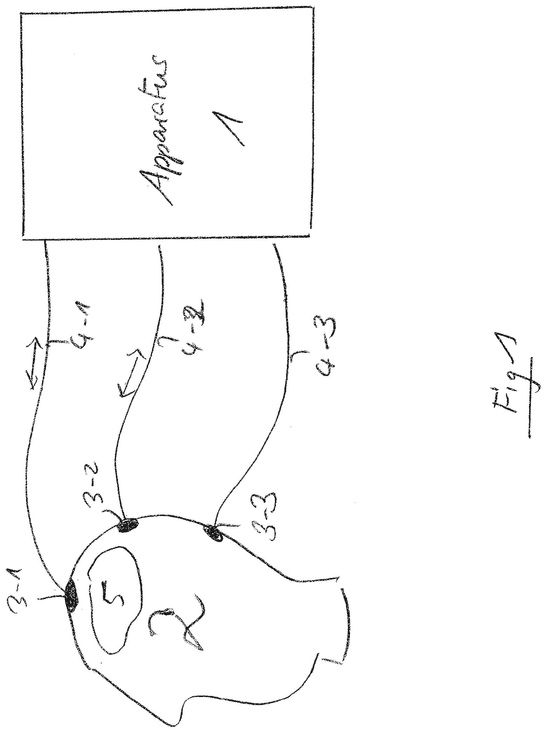

As can be seen from Fig. 1, the apparatus 1 for stimulating a patient according to the present invention is attached to a skull or head of a patient 2 by means of electrodes 3. In the shown embodiment the stimulation apparatus 1 is connected to the head of the patient 2 by means of three electrodes 3-1, 3-2, 3-3. All electrodes 3-i are connected to the stimulation apparatus 1 in the shown embodiment via electrical cables 4-1, 4-2, 4-3. The number of electrodes 3-i and corresponding cables 4-i can vary. In the shown embodiment of Fig. 1 two signal electrodes, i.e. electrodes 3-1, 3-2 are provided for application of an electrical stimulation signal to the head in one operation mode and for deriving an electrical measuring signal from the head comprising a brain 5 in the other operation mode. In the embodiment shown in Fig. 1 a third electrode 3-3 forms a reference electrode providing a reference potential for the stimulation apparatus 1. In the embodiment shown in Fig. 1 the signal electrodes 3-1, 3-2 are formed by so-called dual electrodes which can be switched between two operation modes. In an operation mode of the stimulation apparatus 1 an electrical signal such as an EEG-signal which forms a derived electrical potential from the brain 5 is measured and in another operation mode a generated electrical stimulation signal provided by the stimulation apparatus 1 is applied to the skin of the head of the patient 2.

In the embodiment shown in Fig. 1 electrodes 3-1, 3-2, 3-3 are attached to the surface of the skin of the patient 2. In an alternative non-claimed example the electrodes 3-i can be implanted under the skin of the patient 2.

The number of electrodes 3-i can vary. In other embodiments more than three electrodes are provided.

The location of the electrodes 3-i attachable to the head of the patient 2 can vary depending on the disease or disorder to be treated by the stimulation apparatus 1 according to the present invention.

The stimulation apparatus 1 according to the present invention applies via the electrodes 3-i an electrical stimulation signal having at least one variable stimulation signal parameter such as a signal frequency. This stimulation signal parameter is varied by the stimulation apparatus 1 permanently in a calculated parameter range around a determined neurophysiological fundamental parameter of the patient or around a preset parameter value.

The neurophysiological fundamental parameter can be determined in a possible embodiment of the stimulation apparatus 1 on the basis of a detected or derived electrical signal taken from the head of the patient 2. The detected electrical signal received by the stimulation apparatus 1 via the electrodes 3 in the measuring operation mode is analysed to determine the neurophysiological fundamental parameter of said patient 2 such as a specific neurophysiological fundamental frequency. Around this determined neurophysiological fundamental frequency the stimulation signal is varied in a calculated parameter range. The electrodes 3-i attached to the patient 2 are switchable between the application of the electrical stimulation signal to the head of the patient 2 in the first operation mode and deriving an electrical signal to be recorded from the head of the patient 2 in a second operation mode. This switching of the electrodes 3-i is controlled in a possible embodiment by a control unit integrated in the stimulation apparatus 1. In a possible embodiment the electrical stimulation in the first operation mode and the deriving of an electrical signal to be recorded from the patient 2 in the second operation mode is performed in an alternating manner under the control of the control unit.

In a possible embodiment the stimulation apparatus 1 first derives in a signal measuring operation mode an electrical signal such as an EEG-signal from the brain 5 of the patient 2 and then calculates the individual neurophysiological fundamental parameter such as an individual neurophysiological fundamental frequency of the respective patient 2. Then a parameter range around the determined neurophysiological fundamental parameter is calculated and the stimulation apparatus 1 switches to a signal application operation mode.

After having determined the neurophysiological fundamental parameter and the corresponding parameter range the stimulation apparatus 1 according to the present invention applies in the signal a parameter of application operation mode an electrical stimulation signal which is varied permanently in the respective calculated parameter range around the determined neurophysiological fundamental parameter. In an alternative embodiment the stimulation signal parameter is permanently varied in the calculated parameter range around a preset parameter value input by an operator by means of an operator interface.

For the spontaneous neurological activity of the human brain several frequency ranges are known which can be divided into the delta frequency range (0.5 - 3 Hz), the theta frequency range (4 - 7 Hz), the alpha frequency range (8 - 13 Hz), the beta frequency range (14 - 30 Hz) and the gamma frequency range (31 - 60 Hz).

For the treatment of different disorders of the brain 5 different frequency ranges can be used. For example, for the treatment of visual disorders of the visual cortex system of the brain 5 the alpha frequency range between 8 Hz and 13 Hz is used since it plays an important role in processing of visual impressions. A brain 5 of a patient 2 shows resonance phenomena. If the brain 5 of a patient is stimulated by a stimulation signal having a predetermined stimulation frequency a spontaneous resonance response of the brain 5 can be caused wherein this spontaneous resonance response signal has a frequency which is equal to the stimulation frequency or which has a higher frequency being the product of the stimulation frequency and an integer multiplication factor wherein the applied stimulation frequency is multiplied with this integer multiplication factor or which has a lower frequency being the ratio of the stimulation frequency and a division factor wherein applied the stimulation frequency is divided by this integer number.

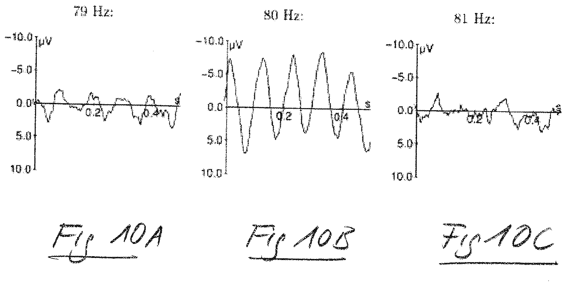

This resonance response is only shown by the brain 5, when the respective brain 5 is stimulated with a stimulation frequency which is relevant for the internal information processing of the brain 2. An example for the resonance phenomena shown by a brain 5 is described in more detail with reference to Fig. 10A, 10B, 10C. The stimulation apparatus 1 according to the present invention for stimulating a person or patient 2 treats one or several disorders of the brain 2 such as visual disorders using this resonance effect for a focused treatment of the respective disorder.

In a possible embodiment the disorder to be treated is input in the stimulation apparatus 1 according to the present invention via a user interface such as a keyboard or a graphical user interface GUI by an operator such as a doctor or a nurse. In this embodiment the stimulation apparatus 1 according to the present invention selects a stored relevant frequency band from a memory of the stimulation apparatus 1 according to the input disorder such as the delta frequency band, the theta frequency band, the alpha frequency band, the beta frequency band or the gamma frequency band.

For example if the operator inputs a visual disorder to be treated, the stimulation apparatus 1 according to the present invention selects in this embodiment the alpha frequency band i.e. a frequency range between 8 and 13 Hz. In this case the neurophysiological fundamental frequency of the patient 2 to be determined is expected to lie in the preselected alpha frequency band i.e. between 8 and 13 Hz. The patient 2 to be treated has his individual neurophysiological fundamental frequency which furthermore can slightly change or drift over time. Accordingly before a stimulation is performed the actual neurophysiological fundamental parameter i.e. the neurophysiological fundamental frequency of the respective patient 2 at this moment is determined.

In a possible embodiment the stimulation apparatus 1 generates an electrical stimulation signal consisting of several periodic electrical stimulation signal sequences each having a corresponding stimulation frequency wherein said electrical stimulation signal sequences are separated by interruptions of a predetermined time period and have different stimulation frequencies within said calculated frequency range, e.g. within the alpha frequency range of 8 to 13 Hz.

The complex brain 5 of the patient 2 comprises different areas and have for fulfilment of complex tasks to be synchronized to each other. In a healthy brain 5 such a synchronization is formed by the brain 5 automatically. In a damaged brain 5 or a brain showing functional disorders, the synchronization between different brain areas does not work properly. The electrical stimulation signal applied by the stimulation apparatus 1 around the neurophysiological fundamental frequency of the patient 2 causes a resynchronization of the damaged brain areas. By permanently varying the stimulation signal parameter, such as a frequency, it can be avoided that the brain 5 of the patient 2 gets used (adapts) to the external synchronization frequency which would prevent the brain resynchronization effect.

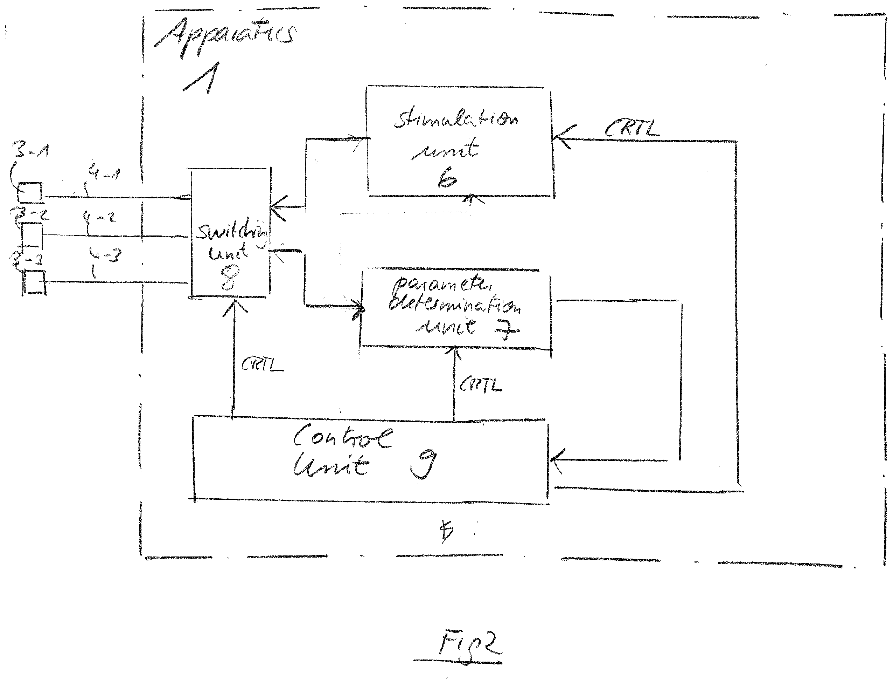

Fig. 2 shows an embodiment of the stimulation apparatus 1 according to the present invention. In the embodiment shown in Fig. 2 the stimulation apparatus 1 for treatment of the patient 2 comprises a stimulation unit 6 and a parameter determination unit 7. The stimulation unit 6 and the parameter determination unit 7 are both connected to a switching unit 8. The stimulation unit 6, the parameter determination 7 as well as the switching unit 8 are controlled via control lines by a control unit 9 integrated in the stimulation apparatus 1 as shown in Fig. 2. The stimulation unit 6 is provided for generating an electrical stimulation signal which in a possible embodiment can consist of several periodic electrical stimulation signal sequences each having a corresponding stimulation frequency.

The parameter determination unit 7 shown in Fig. 2 is provided for determining at least one neurophysiological fundamental parameter. The neurophysiological fundamental parameter can be the neurophysiological fundamental frequency of the patient 2 as recorded by an electroencephalogram (EEG) or by a magnet-encephalogram.

The electrodes 3-i attached to the head 2 of the patient are switchable by means of the switching unit 8 between the application of an electrical stimulation signal generated by the stimulation unit 6 to the patient 2 in a first operation mode and deriving signal to be recorded from the head of the patient 2 and evaluated by the parameter determination unit 7 in a second operation mode. Switching of the electrodes 3-i by means of the switching unit 8 is controlled by the control unit 9 as shown in Fig. 2.

In a possible embodiment the parameter determination unit 7 can comprise recording means for recording an EEG-signal taken from the head of the patient 2 and transforming means for transforming the recorded EEG-signal into the individual neurophysiological fundamental frequency of said patient in the respective moment.

In a possible embodiment this transforming means of the parameter determination unit 7 is formed by a Fourier Frequency Transformation means adapted to perform a Fourier Frequency Transformation of the recorded EEG-signal.

In an alternative embodiment the transforming means is formed by auto regression means which are adapted to perform an auto regression of the recorded EEG-signal.

In a possible embodiment the recording of the EEG-signal of the patient 2 by the recording means of the parameter determination unit 7 is performed for a predetermined recording time period with the eyes of the patient 2 closed before the electrical stimulation of the patient 2 is performed by applying the electrical stimulation signal generated by the stimulation unit 6.

In a possible embodiment the stimulation unit 6 varies the stimulation frequency of the electrical stimulation signal continuously.

In an alternative embodiment the stimulation unit 6 varies a stimulation frequency of the electrical stimulation signal applied to the patient 2 discretely.

The control unit 9 is provided for controlling the determination of the neurophysiological fundamental parameter of the respective patient 2 by the detection unit 7 and for controlling the stimulation of the patient 2 with an electrical stimulation signal generated by the stimulation unit 6. In a possible embodiment the determination of the neurophysiological fundamental parameter such as the neurophysiological frequency of the patient 2 and the treatment of the patient 2 with a stimulation signal are controlled by a control unit 9 to be performed in an alternating manner.

In a possible embodiment the apparatus 1 comprises at least one memory to store the signals recorded by means of the electrodes 3-i attached to the patient 2. This memory can be located in the parameter determination unit 7.

In a possible embodiment the control unit 9 can be formed by a microprocessor executing a computer program which controls the stimulation unit 6 and the parameter determination unit 7.

In a further embodiment of the apparatus 1 according to the present invention, the parameter determination unit 7 is adapted to detect a flicker-fusion frequency as the neurophysiological fundamental frequency of the patient 2. In this embodiment the stimulation unit 6 stimulates the patient 2 with an electrical stimulation signal having an adjustable stimulation frequency which is increased to cause a rhythmic flickering light sensation of the patient 2 until the stimulation frequency causes a constant light sensation of the patient 2. This adjusted stimulation frequency forms the flicker-fusion frequency of the patient 2 which forms a neurophysiological fundamental frequency of the patient 2. In this embodiment the frequency of the stimulation signal can be increased slowly from a relatively low frequency value of e.g. 4 Hz to a relatively high frequency of e.g. 40 Hz. Normally a patient 2 has an optical impression called phoshenes which flicker in the rhythm of the electrical stimulation frequency. The frequency of the stimulation signal is increased up to a certain threshold frequency when the flickering light sensation ends and the light sensation is recognized by the patient 2 as a constant illumination. This is the so-called "flicker-fusion frequency" having a typical value within the range of 16- 40 Hz depending on the individual patient 2. From the neurological point of view this frequency forms an important limit for the processing velocity of the brain 5 responding to visual impressions or stimulations.

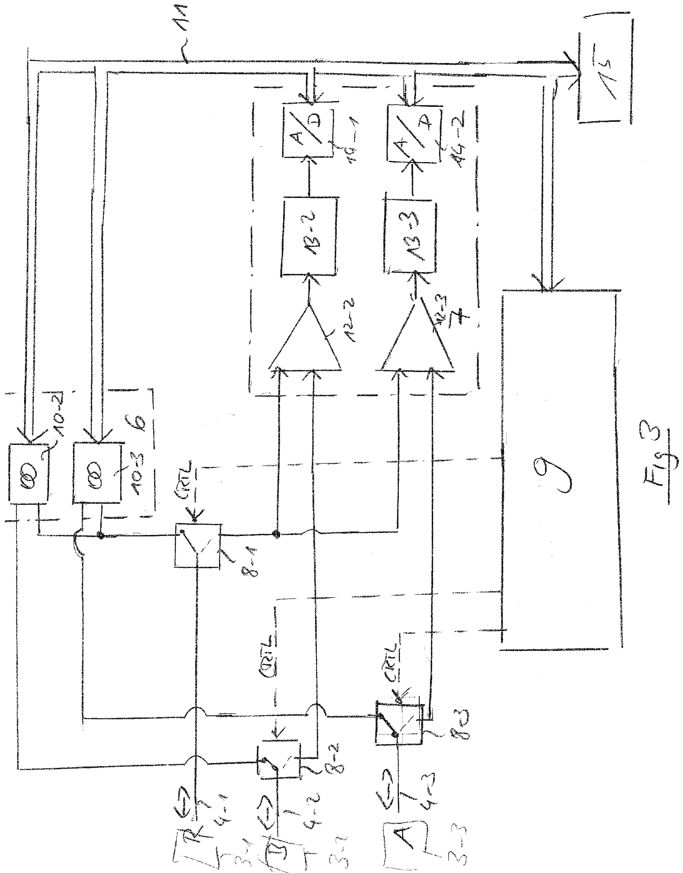

Fig. 3 shows a possible embodiment of the stimulation apparatus 1 according to the present invention for stimulating a patient 2. In this embodiment the switching unit 8 shown in Fig. 2 consists of three switches 8-1, 8-2, 8-3 each controlled by the control unit 9 between two positions. In the block diagram of Fig. 3 the electrodes 3-1, 3-2, 3-3 are switched to the stimulation unit 6 and receive an electrical stimulation signal generated by the stimulation unit 6. In the other position of the switches 8-1, 8-2, 8-3 the electrodes 3-1, 3-2, 3-3 are switched to the parameter determination unit 7 for evaluating the detected electrical signal for determination of at least one neurophysiological fundamental parameter of the respective patient 2. The switches 8-i shown in Fig. 3 can be mechanical switches such as reed relays or semiconductor switches such as MOSFETs or bipolar transistors.

In the embodiment of the apparatus 1 according to the present invention as shown in Fig. 3 the stimulation unit 6 comprises controllable current sources 10-2, 10-3 connected to the control unit 9 by means of a digital data bus 11. The stimulation unit 6 comprises for each signal electrode 3-2, 3-3 a corresponding adjustable current source 10-i. For the reference electrode 3-1 no controllable current source is necessary. The controllable current sources 10-2, 10-3 are controlled by the control unit 9 via the internal data bus 11 and receive commands for generating of stimulation current pulses.

The parameter determination unit 7 shown in the embodiment of fig. 3 comprises for each signal electrode 3-2, 3-3 a corresponding analogue signal preamplifier 12-2, 12-3 which can be formed by an operation amplifier. The preamplifiers 12-2, 12-3 amplify a signal between the respective signal electrode and the reference electrode in the second operation mode for evaluating the derived signals. The output of each analogue preamplifier 12-2, 12-3 is connected to an analogue filter 13-2, 13-3. In a possible embodiment the analogue filters 13-i are formed by band pass filters. In a possible embodiment artefacts such as movements of the eyes or the head of the patient 2 are removed from the detected signal. Furthermore, highfrequency noise (e.g. above 100 Hz) is also removed. The output of the analogue filters 13-i is connected to a corresponding analogue-/digital converter 14-1, 14-2 as can be seen in Fig. 3. The converted signal can be provided via the digital bus 11 to the control unit 9 as shown in fig. 3. In the shown embodiment of Fig. 3 the control unit 9 performs a data processing of the converted electrical signal taken from the brain 5 of the patient 2 and evaluates this signal to determine a neurophysiological fundamental parameter of the patient 2. Accordingly in the embodiment of Fig. 3 the control unit 9 performs part of the functions of the parameter determination unit 7.

In an alternative embodiment the parameter determination unit 7 does not only comprise analogue preamplifiers 12-i, filters 13-i and analogue-/digital converters 14-i but also recording means for recording the technical electrical signal and transforming means for transforming the recorded electrical signal to determine the neurophysiological fundamental frequency of the patient. In this alternative embodiment the parameter determination unit 7 comprises at least one processor of its own.

In a possible embodiment for each signal electrode 3-2, 3-3 a separate signal processor can be provided for calculating neurophysiological fundamental frequencies of the patient 2 and then an average value of all calculated neurophysiological fundamental frequencies is taken and forwarded to the control unit 9 for controlling the stimulation unit 6.

In a possible embodiment the transforming means of the determination unit 7 forms a Fourier Frequency Transformation of the recorded signal.

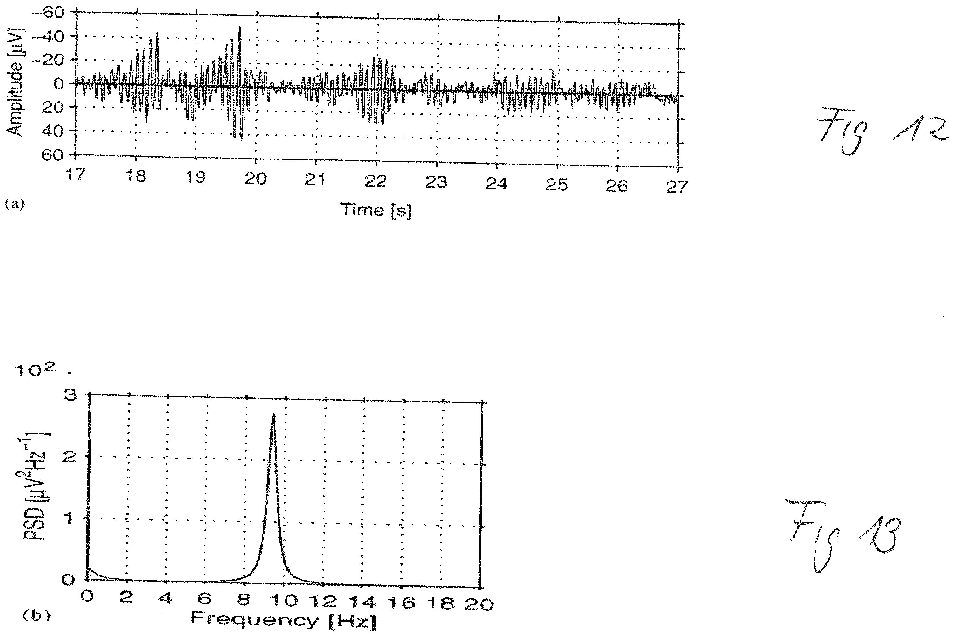

In an alternative embodiment the transforming means of the parameter determination unit 7 performs an auto regression of the recorded signal. Fig. 12 shows an example of a recorded EEG-signal having frequencies in the alpha frequency range. The transforming means of the determination unit 7 performs a Fourier Frequency Transformation or an auto regression to calculate a fundamental frequency of the patient 2. In the given example an EEG-signal as shown in Fig. 12 the transforming means of the parameter determination unit 7 for example calculates a fundamental frequency of e.g. 10 Hz as shown in the frequency spectrum shown in Fig. 13. The calculated frequency spectrum has a peak at 10 Hz as can be seen in Fig. 13 forming the neurophysiological fundamental frequency of the individual patient in this moment. The determined fundamental neurophysiological frequency is individual i.e. two different patients have two different neurophysiological fundamental frequencies. Furthermore, the neurophysiological fundamental frequency of a patient 2 is not constant but can vary over time when it is determined again after each applied stimulation sequence.

In a possible embodiment the common digital data bus 11 of the stimulation apparatus 1 connects the current sources 10-1, the outputs of the analogue digital converters 14-i and the control unit 9. Furthermore an interface 15 can be provided connecting the stimulation apparatus 1 to an external computer. This interface 15 can be formed by an USB-interface.

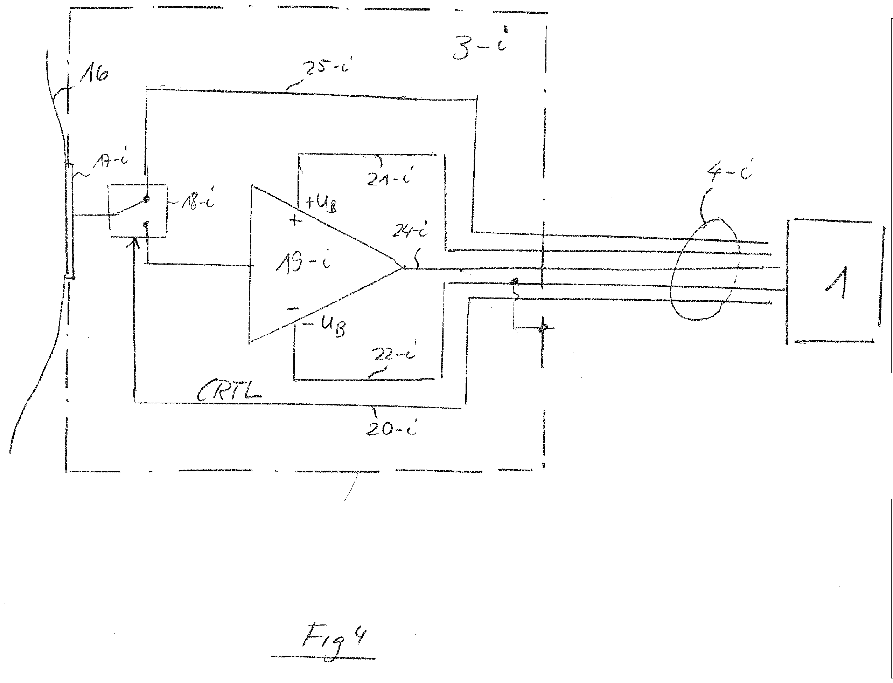

Fig. 4 shows a diagram of an electrode 3-i as employed by the stimulation apparatus 1 according to the present invention in a preferred embodiment. The electrode 3-i shown in Fig. 4 is a dual-electrode attachable to a skin 16 at the head of the patient 2 to be treated. The dual electrode 3-i as shown in Fig. 4 comprises a passive electrode plate 17-i attached to the skin 16. This passive electrode plate 17 is formed by a suitable material such as Ag/Cl. Within the dual electrode 3-i an electronic switch 18-i is provided which can be controlled by the remote control unit 9 of the stimulation apparatus 1 via a control line 20-i within the cable 4-i. The dual electrode 3-i comprises an analogue signal amplifier 19-i of its own which is supplied with power by the stimulation apparatus 1 via power lines 21-i, 22-i within the cable 4-i. The output of the first signal amplifier 19-i within the dual electrode 3-i is connected via a signal line 24-i of the cable 4-i in the detection and signal evaluation mode to the input of the second analogue signal amplifier 12-i within the stimulation apparatus 1 as shown for example in Fig. 3. In the embodiment shown in Fig. 4 the cable 4-i connecting the dual electrodes 3-i with the stimulation apparatus 1 comprises at least five lines and each line can be shielded electrically by means of suitable material such as a metal. Further a coating can protect the cable against touching wherein the coating can be formed by an electrically isolating material.

The cable 4-i comprises in the shown embodiment two power lines (+UB -UB) 21-i, 22-i supplying the preamplifier 14-i integrated within the dual electrode 3-1, a signal line 24-i for transmitting the preamplified recorded signal such as an EEG-signal to the parameter determination unit 7 within the apparatus 1, a control line 20-i for switching the integrated electronic switch 18-i between the two operation modes and a stimulation signal line 25-i for applying the generated stimulation signal via the switch 18-i to the passive electrode plate 17-i of the dual electrode 3-i.

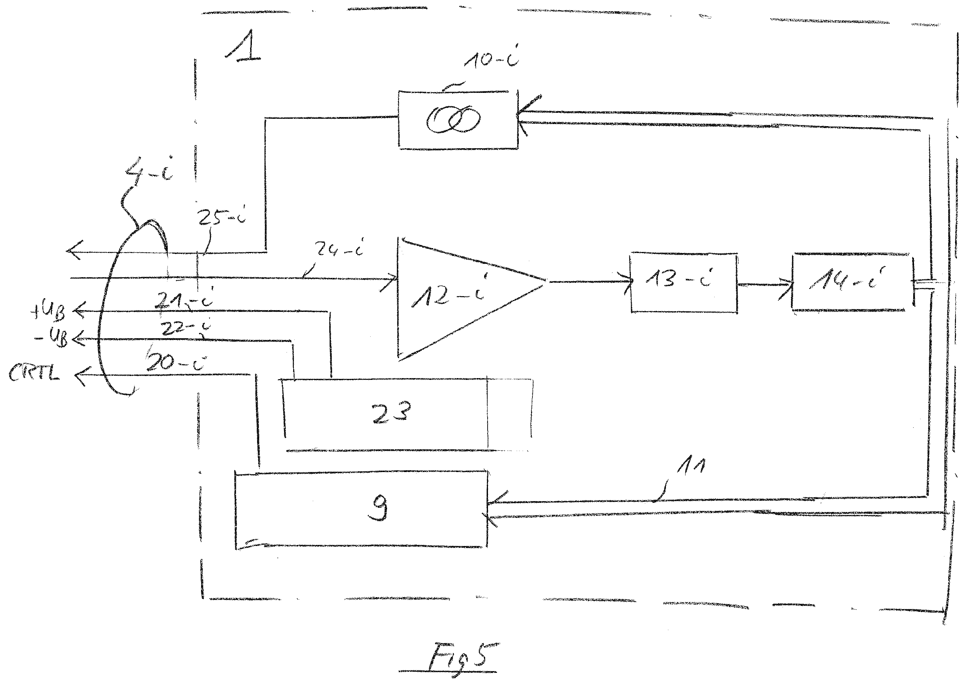

Fig. 5 illustrates the connection of different components within the stimulation apparatus 1 to an active dual electrode 3-i as shown in fig. 4.

The control line 20-i is connected to the control unit 9 of the stimulation apparatus 1. The power supply lines 21-i, 22-i are connected to a power supply unit 23 within the stimulation apparatus 1. The signal line 24-i for transmitting the detected signal such as the EEGsignal is connected to the preamplifier 12-i within the stimulation apparatus 1 via a switch 8-i as shown in Fig. 4.

In the embodiment shown in Fig. 4, Fig. 5 each dual electrode 3-i is connected to the stimulation apparatus 1 via a corresponding cable 4-i each comprising five signal lines. In an alternative embodiment all dual electrodes 3-i are connected to the stimulation apparatus 1 via a common single cable.

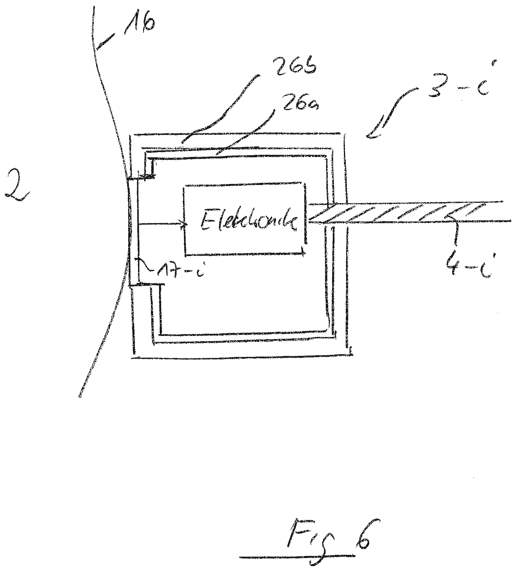

Fig. 6 shows a further diagram illustrating a possible embodiment by a dual electrode 3-i as employed by the stimulation apparatus 1 according to a possible embodiment. In this embodiment the electronic circuits of the dual electrode 3, i.e. the semiconductor switch 18-i and the preamplifier 19-i are integrated in a housing 26 consisting of a shielding 26A and an electrical isolation 26B. The passive electrode material of the plate 17-i can be formed by gold or Ag/Cl.

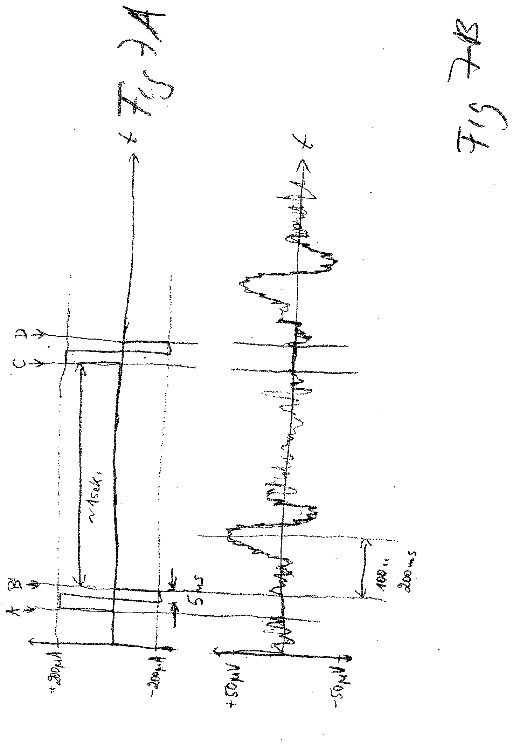

Fig. 7A, 7B show signal diagrams for illustrating a possible embodiment of a stimulation apparatus 1 according to the present invention.

The stimulation unit 6 of the apparatus 1 generates an electrical stimulation signal an example of which is shown in Fig. 7A. In an exemplary embodiment the current strength of the applied signal lies in a range of 200 μA and the pulse width within the pulse train is in a range of 5 to 10 ms. Each single impulse can be followed by an interruption period in the range of one second.

In the signal diagram of Fig. 7B a typically measured EEG-signal is shown. Typical signal amplitudes are within a range of +/- 50 μV.

Each single impulse of the stimulation signal as shown in Fig. 7A causes in the brain 5 of the patient 2 reactions depending on the applied amplitude, duration and signal form of the applied stimulation signal. In the diagram shown in Fig. 7A at A, C the stimulation unit 6 is switched to the electrodes 3 and the parameter determination unit 7 is separated. At B, D the stimulation unit 6 is switched off and the electrodes 3 are connected to the parameter determination unit 7. As shown in Fig. 7A each impulse can be formed by a positive signal peak followed by a negative signal peak causing an integral value of zero.

As shown in Fig. 7B because of the cognitive processes within the brain 5 caused by the stimulation signal impulses the signal response of the brain 5 is generated with a certain time delay of e.g. 100 to 200 msec. The signal response derived from the brain 5 can have a positive response pulse or a negative response pulse as shown in Fig. 7B.

In a preferred embodiment the stimulation signal has a significantly higher amplitude than the derived response or EEG-signal.

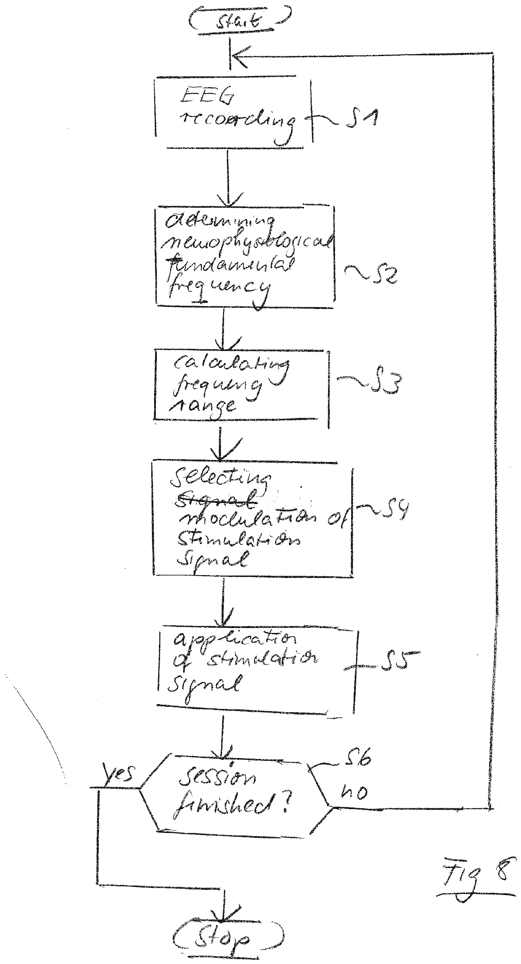

Furthermore Fig. 8 shows a possible non-claimed example of a method for stimulating a patient with an electrical stimulation signal. For treating the patient 2 with a stimulation signal first a recording of an EEG-signal is performed in step S1 to evaluate the actual physical neurological state of the patient 2. This deriving and recording of the EEG-signal can be performed for a predetermined time period with the eyes of the patient 2 closed. In a possible embodiment this predetermined recording time period can last 1 to 2 minutes.

After recording of the EEG-signal in step S 1 the neurophysiological fundamental frequency is determined in step S2 by the parameter determination unit 7 within the stimulation apparatus 1. For example, with the EEG-signal as shown in Fig. 12 a fundamental physiological frequency of the patient of 10 Hz is determined as shown in Fig. 13. In a possible embodiment the determination of the neurophysiological fundamental frequency is done by transforming means which performs a Fourier Frequency Transformation or an auto regression AR.

In a possible non-claimed example the determination of the neurophysiological fundamental frequency is performed after signal artefacts caused for example by movement of the eyes or the head of the patient 2 has been extracted from the signal.

In a further step S3 a frequency range around the determined neurophysiological fundamental parameter e.g. around the fundamental frequency is calculated. In a possible embodiment this is done by multiplying the detected neurophysiological fundamental frequency by a upper and lower weighting factor indicating for example a percentage value. The upper weighting or limitation factor might be 140% = 1.4 and the lower weighting factor might be 80% = 0.8. If the neurophysiological fundamental frequency determined in step S2 is, for example, 10 Hz this leads to an upper frequency limit of 14 Hz and a lower frequency limit 8 Hz. Accordingly the calculated parameter range, e.g. the frequency range of 8 - 14 Hz.

In a further step S4 a desired signal modulation of the stimulation signal can be selected. In one embodiment the stimulation frequency or parameter of the stimulation signal is varied discretely. In this embodiment the frequency range between the upper frequency limit and the lower frequency limit can be divided in up to n-ranges of similar size. For example with n = 5 the following frequencies are calculated:

8 Hz, 9,5 Hz, 11 Hz; 12,5 Hz; 14 Hz.

Starting from the lowest frequency, i.e. 8 Hz the complete calculated frequency range is covered by sweeping through in discrete steps. After reaching the highest frequency i.e. 14 Hz the complete calculated frequency range is swept through in a reverse order. Accordingly a stimulation signal with varying frequency is applied to the patient 2 to be treated, i.e. the stimulation signal has a varying frequency of 8 Hz, 9,5 Hz, 11 Hz, 12,5 Hz, 14 Hz, 12,5 Hz, 11 Hz, 9,5 Hz, 8 Hz, 9,5 Hz.... etc.

In an alternative non-claimed example the calculated frequency range runs through continuously e.g. the stimulation unit 6 of the stimulation apparatus 1 varies the stimulation frequency continuously between both frequency limits.

In a further non-claimed example a signal with frequencies varying within the calculated frequency range is applied by the stimulation unit 6 of the stimulation apparatus 1 at random.

In a further non-claimed example the frequency range between the lower and upper frequency range is divided up to 4-ranges of similar size to have serial discrete frequency steps such as 8 HZ, 9,5 HZ etc. and then the frequency steps are selected at random by means of a random generator.

All examples have in common that the brain 5 of the patient 2 cannot get used to a specific stimulation frequency because the stimulation frequency is varied by sweeping through a predetermined frequency range or by picking a frequency within the calculated frequency range at random.

In a further step S5 the stimulation signal is applied by the stimulation unit 6 to the skin of the head of the patient 2 to be treated. For example, the patient 2 can be stimulated with the stimulation frequency electrically for a predetermined time period lasting, for example, 1 - 20 seconds. In this embodiment the stimulation unit 6 generates an electrical stimulation signal consisting of several periodic electrical stimulation signal sequences each having a corresponding stimulation frequency. These electrical stimulation signal sequences can be separated by signal interruptions of a predetermined time period and have different stimulation frequencies within the calculated frequency range.

In a possible non-claimed example the electrical stimulation signal sequences of the electrical stimulation signal have the same signal form. In an alternative non-claimed example the electrical stimulation signal sequences of the electrical stimulation signal can comprise different signal forms as well.

The periodic electrical stimulation signal sequences can comprise pulses or have a single sinusoidal signal waveform.

In a possible non-claimed example a pulse length of a pulse within an electrical stimulation signal sequence is decreased with increasing stimulation frequency of the electrical stimulation signal sequence. Accordingly, a modification of the impulse width can be performed proportionally to the frequency change.

The frequency is the product of a multiplication factor multiplied with the fundamental frequency.

The adapted pulse width is a predetermined initial pulse width divided by the multiplication factor.

In an embodiment the factor is e.g. 1.2 and the patient 2 has a main or fundamental frequency of 10 Hz. The given initial impulse width is e.g. 9 ms. With this given frequency the adapted pulse width can be calculated as follows:

$$\text{Stimulation Frequency} = 1.2 \times 10 \text{ Hz} = 12 \text{ Hz}$$

and

$$\text{Impulse width} = 9 \text{ ms} : 1.2 = 7.5 \text{ ms}$$

For example, starting from a detected fundamental frequency determined in step S2 of e.g. 10 Hz and a set multiplication factor of 0.8 and 1.4 corresponding to a calculated frequency range between 8 Hz and 14 Hz the modulation frequency is varied in a possible non-claimed example in concrete equidistant steps between 8 Hz, 9,5 Hz, 11 Hz, 12,5 Hz and 14 Hz. In a possible embodiment the patient 2 is first stimulated with a frequency of 8 Hz with a sinusoidal signal for 10 seconds followed by a signal interruption of 1 minute.

After that the stimulation unit 6 applies a stimulation signal with a frequency of 9.5 Hz with a sinusoidal waveform for 10 seconds followed again by a signal interruption of 1 minute.

Then the stimulation unit 6 applies the next frequency within the frequency range, i.e. a frequency of 11 Hz and for 10 seconds followed by a signal interruption of 1 minute and so on.

After having reached the maximum frequency of 14 Hz the stimulation signal frequency is diminished in calculated frequency steps.

In an alternative non-claimed example frequencies are selected at random from the calculated frequency range between 8 Hz and 14 Hz, e.g. 8 Hz, 8,3 Hz, 9,7 Hz, 13,1 Hz, 9,2 Hz etc.

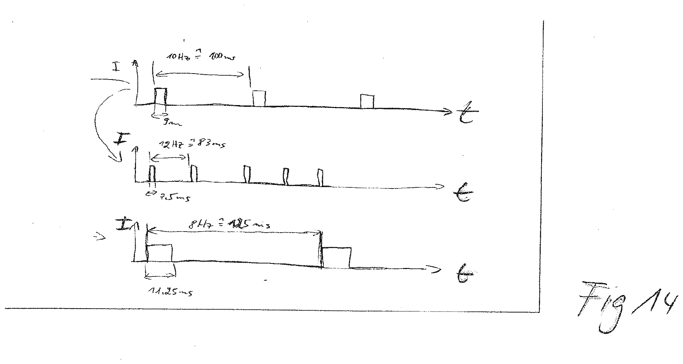

In an alternative non-claimed example as illustrated by Fig. 14 the electrical stimulation is performed with adapted impulse width. For example a stimulation signal with a frequency of 10 Hz having a pulse width of 9 ms followed by a stimulation signal having a frequency of 12 Hz with a reduced pulse width of 7.5 ms followed by a stimulation signal having a frequency of 14 Hz and a further reduced impulse width of 6.42 ms etc.

In the non-claimed example of Fig. 8 an electric stimulation is performed with a feedback.

After a predetermined time of stimulation an EEG-signal is recorded and evaluated to detect a possibly changed fundamental frequency of the brain 5.

In the non-claimed example shown in Fig. 8 in step S6 it is decided whether the treatment or session is finished. When the overall treatment time is reached the stimulation is stopped. Otherwise the process returns to step S1.

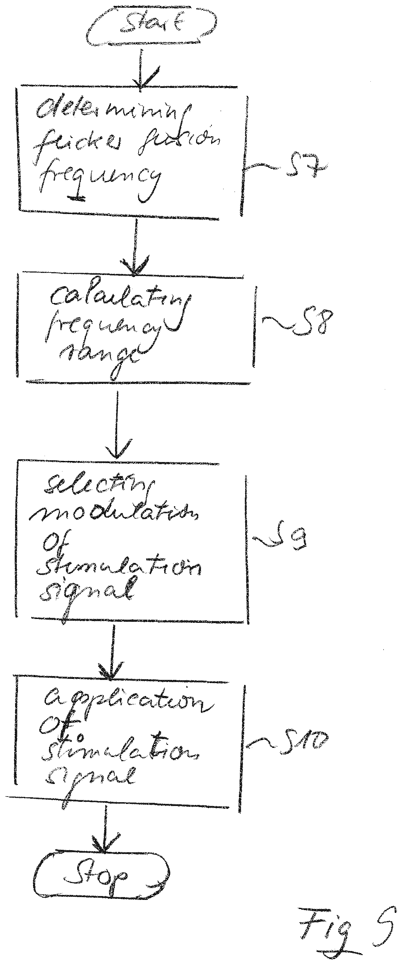

Fig. 9 shows a flow chart of an alternative non-claimed example of a method for stimulation of a patient 2 performed by a stimulation apparatus 1 according to the present invention.

In this non-claimed example the stimulation apparatus 1 first determines a so-called flicker-fusion frequency in step S7. The stimulation unit 6 stimulates the patient with the electrical stimulation signal having an adjustable stimulation frequency which is increased to cause a rhythmic flickering light sensation of the patient 2 until the stimulation frequency causes a constant light sensation of the patient 2. This adjusted stimulation frequency at the sensation change forms the flicker-fusion frequency being a neurophysiological fundamental frequency of the respective patient 2.

On the basis of the determined flicker-fusion frequency a frequency range is calculated in step S8 similar to step S3 of the embodiment shown in Fig. 8. In a further step S9 corresponding to step S4 shown in Fig. 8 the modulation of the stimulation signal is selected.

After that the stimulation signal is applied in step S10 corresponding to step S5 of the non-claimed example shown in Fig. 8. As can be seen from the flow chart shown in Fig. 9 in this non-claimed example there is no feedback. In the non-claimed example with no feedback as shown in Fig. 9 the electrical stimulation treatment of the patient 2 is performed within a frequency range being calculated once.

Fig. 10A, 10B, 10C show signal diagrams illustrating a frequency resonance phenomena of a brain. In the given example the brain 5 of a patient 2 is stimulated with different frequencies e.g. the frequency of 79 Hz as shown in Fig. 10A, with a frequency of 80 Hz as shown in Fig. 10B and with a frequency of 81 Hz as shown in Fig. 10c. When applying a stimulation signal having a frequency of 80 Hz, the brain 5 generates a neurophysiological fundamental response frequency of 80 Hz as shown in the signal diagram of Fig. 10B.

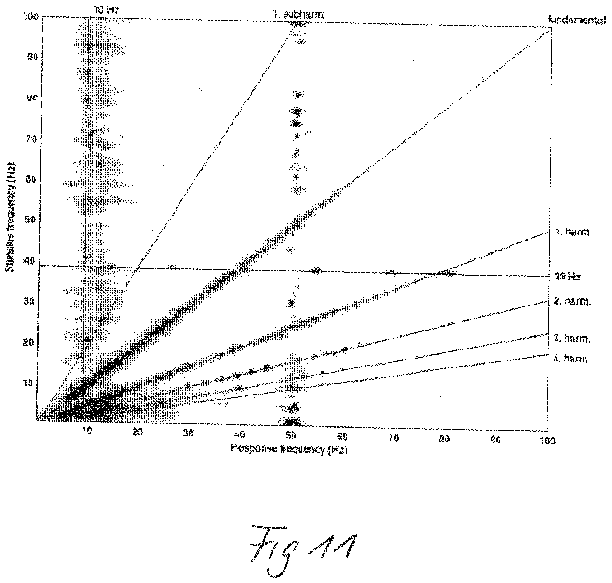

Fig. 11 is a further diagram to illustrate such a resonance frequency phenomen shown by a patient 2. In this example the stimulation frequency can change between 1 Hz and 100 Hz. The vertical y axis of the diagram shown in Fig. 11 indicates the stimulation frequency changing from 1 to 100 Hz. The horizontal x-axis of the diagram shown in Fig. 11 indicates a measured response frequency. As can be seen from the diagram in Fig. 11 the brain 5 shows a resonance effect at certain frequencies i.e. at a stimulation frequency of 39 Hz and there are resonance response frequencies of 13 Hz, 26 Hz, 39 Hz, 52 Hz, 59 Hz and 81 Hz.

The stimulation apparatus 1 according to the present invention can be used for treatment of a variety of disorders caused by brain damages or brain diseases of a patient 2. In particular the stimulation apparatus 1 according to the present invention can be used to treat language disorders, cognitive impairments, movement disorders, memory disorders as well as auditory and visual disorders of the patient. Further the apparatus 1 according to the present invention can be used to treat functional neurological disorders of a patient 2. Other possible diseases to be treated by the apparatus 1 of the present invention are diseases of the retina of an eye of the patient 2. Further epilepsy and depression disorders of the patient 2 can be treated. Also amblyoia can be treated.

Further examples of a stimulation apparatus 1 according to the present invention are possible. For example, the stimulation apparatus 1 can also use conventional electrodes instead of dual electrodes. In this embodiment the stimulation signal is applied via a first set of signal electrodes and the recorded signal is derived by means of another set of signal electrodes.

Besides the electrical stimulation via the electrodes 3 the patient can additionally be stimulated by visual or auditory stimulation signals which have in a possible example the same frequency as the electrical stimulation signal. For example, a patient 2 to be treated can receive an electrical stimulation signal with stimulation frequencies varied in a calculated frequency range around a determined neurophysiological frequency and simultaneously look at a display providing a video signal having the same frequency as the electrical signal and having the same signal phase.

**Claims**

1. An apparatus (1) for stimulating a patient (2), comprising:

   a stimulation unit (6) for applying an electrical stimulation signal having at least one variable stimulation signal parameter, wherein said stimulation signal parameter is permanently varied in a calculated parameter range around a determined neurophysiological fundamental parameter of said patient (2),
   wherein a parameter determination unit (7) is provided for determining at least one neurophysiological fundamental parameter, wherein said parameter determination unit (7) comprises means for detecting an electrical signal from the brain (5) of said patient (2) and means for analyzing said detected electrical signal to determine said neurophysiological fundamental parameter of said patient (2),
   wherein said determined neurophysiological fundamental parameter comprises a neurophysiological fundamental frequency of said patient (2), wherein said parameter determination unit (7) comprises electrical electrodes (3) configured to be attached to the surface of the skin of said patient (2), wherein the electrodes (3) are adapted to apply the electrical stimulation signal and to detect an electrical signal from the brain (5).

2. The apparatus according to claim 1,

   wherein said stimulation unit (6) generates an electrical stimulation signal consisting of several periodic electrical stimulation signal sequences each having a corresponding stimulation frequency,
   wherein said electrical stimulation signal sequences are separated by signal interruptions of a predetermined time period and have different stimulation frequencies within said calculated frequency range.

3. The apparatus according to claims 1 to 2,

wherein said parameter determination unit (7) comprises

    - recording means for recording an EEG-signal of said patient (2) and
    - transforming means for transforming the recorded EEG-signal into said neurophysiological fundamental frequency of said patient (2).

4. The apparatus according to claim 3,
wherein said transforming means is formed by Fourier Frequency Transformation means adapted to perform a Fourier Frequency Transformation of said recorded EEG-signal or by autoregression means adapted to perform an auto-regression of said recorded EEG-signal.

5. The apparatus according to claim 1,
wherein said parameter determination unit (7) is adapted to detect a flicker-fusion frequency as the neurophysiological fundamental frequency of said patient (2).

6. The apparatus according to claims 3 to 5,
wherein
the electrodes (3) being adapted to apply an electrical stimulation signal to said patient (2) and to record an EEG-signal of said patient (2).

7. The apparatus according to claim 5,

    wherein said stimulation unit (6) stimulates said patient (2) with the electrical stimulation signal having an adjustable stimulation frequency which is increased to cause a rhythmic flickering light sensation of said patient (2) until said stimulation frequency causes a constant light sensation of the patient (2),
    wherein the adjusted stimulation frequency is the flicker fusion frequency forming said neurophysiological fundamental frequency of said patient (2).

8. The apparatus according to claims 1 to 7,
wherein said stimulation unit (6) varies the stimulation frequency of said electrical stimulation signal continuously or discretely.

9. The apparatus according to claims 1 to 8,

    wherein a control unit (9) is provided for controlling a determination of said neurophysiological fundamental parameter of said patient (2) by the parameter detection unit (7) and for controlling the stimulation of the patient (2) with an electrical stimulation signal by said stimulation unit (6),
    wherein the determination of said neurophysio logical fundamental parameter and the stimulation are controlled by said control unit (9) to be performed in an alternating manner.

10. The apparatus according to claims 2 to 9,
wherein the electrical stimulation signal sequences of said electrical stimulation signal comprise different signal forms.

11. The apparatus according to claim 10,
wherein said periodic electrical stimulation signal sequences comprise pulses or have a sinusoidal signal waveform.

12. The apparatus according to claim 11,
wherein the pulse length of a pulse within an electrical stimulation signal sequence is decreased with increasing stimulation frequency of said electrical stimulation signal sequence.

13. The apparatus according to claims 9 to 12,

    wherein said electrodes (3) attached to said patient (2) are switchable between the application of an electrical stimulation signal to said patient (2) in a first operation mode and deriving a signal to be recorded from said patient (2) in a second operation mode,
    wherein the switching of said electrodes (3) is controlled by said control unit (9).

14. The apparatus according to claims 13,

wherein a memory is provided to store the signals recorded by means of said attached electrodes (3) in said second operation mode.

**Patentansprüche**

1. Vorrichtung (1) zum Stimulieren eines Patienten (2), umfassend:

   eine Stimulationseinheit (6), um ein elektrisches Stimulationssignal mit mindestes einem variablen Stimulationssignalparameter anzulegen, wobei der Stimulationssignalparameter permanent in einem berechneten Parameterbereich um einen bestimmten neurophysiologischen grundlegenden Parameter des Patienten (2) herum variiert wird, wobei eine Parameterbestimmungseinheit (7) vorgesehen ist, um mindestens einen neurophysiologischen grundlegenden Parameter zu bestimmen, wobei die Parameterbestimmungseinheit (7) ein Mittel, um ein elektrisches Signal aus dem Gehirn (5) des Patienten (2) zu erfassen, und ein Mittel umfasst, um das erfasste elektrische Signal zu analysieren, um den neurophysiologischen grundlegenden Parameter des Patienten (2) zu bestimmen, wobei der bestimmte neurophysiologische grundlegende Parameter eine neurophysiologische grundlegende Frequenz des Patienten (2) umfasst, wobei die Parameterbestimmungseinheit (7) elektrische Elektroden (3) umfasst, die dazu ausgelegt sind, an der Oberfläche der Haut des Patienten (2) angebracht zu werden, wobei die Elektroden (3) dazu angepasst sind, das elektrische Stimulationssignal anzulegen und ein elektrisches Signal aus dem Gehirn (5) zu erfassen.

2. Vorrichtung nach Anspruch 1,

   wobei die Stimulationseinheit (6) ein elektrisches Stimulationssignal generiert, das aus mehreren periodischen elektrischen Stimulationssignalsequenzen mit jeweils einer entsprechenden Stimulationsfrequenz besteht, wobei die elektrischen Stimulationssignalsequenzen durch Signalunterbrechungen eines vorbestimmten Zeitraums getrennt sind und verschiedene Stimulationsfrequenzen innerhalb des berechneten Frequenzbereichs haben.

3. Vorrichtung nach den Ansprüchen 1 bis 2,
   wobei die Parameterbestimmungseinheit (7)

   - ein Aufzeichnungsmittel zum Aufzeichnen eines EEG-Signal des Patienten (2), und
   - ein Umwandlungsmittel, um das aufgezeichnete EEG-Signal in die neurophysiologische grundlegende Frequenz des Patienten (2) umzuwandeln, umfasst.

4. Vorrichtung nach Anspruch 3,
   wobei das Umwandlungsmittel durch ein Fourier-Frequenztransformationsmittel, das dazu angepasst ist, eine Fourier-Frequenztransformation an dem aufgezeichneten EEG-Signal vorzunehmen, oder durch ein Autoregressionsmittel gebildet ist, das dazu angepasst ist, eine Autoregression an dem aufgezeichneten EEG-Signal vorzunehmen.

5. Vorrichtung nach Anspruch 1,
   wobei die Parameterbestimmungseinheit (7) dazu angepasst ist, eine Flimmerverschmelzungsfrequenz als die neurophysiologische grundlegende Frequenz des Patienten (2) zu erfassen.

6. Vorrichtung nach den Ansprüchen 3 bis 5,
   wobei
   die Elektroden (3) dazu angepasst sind, ein elektrisches Stimulationssignal an den Patienten (2) anzulegen und ein EEG-Signal des Patienten (2) aufzuzeichnen.

7. Vorrichtung nach Anspruch 5,
   wobei die Stimulationseinheit (6) den Patienten (2) mit dem elektrischen Stimulationssignal stimuliert, das eine einstellbare Stimulationsfrequenz hat, die erhöht wird, um eine rhythmische Flimmerlichtempfindung des Patienten (2) zu bewirken, bis die Stimulationsfrequenz eine konstante Lichtempfindung des Patenten (2) bewirkt, wobei es sich bei der eingestellten Stimulationsfrequenz um die Flimmerverschmelzungsfrequenz handelt, welche die neurophysiologische grundlegende Frequenz des Patienten (2) bildet.

**8.** Vorrichtung nach den Ansprüchen 1 bis 7,
wobei die Stimulationseinheit (6) die Stimulationsfrequenz des elektrischen Stimulationssignals stetig oder unstetig variiert.

**9.** Vorrichtung nach den Ansprüchen 1 bis 8,

wobei eine Steuereinheit (9) vorgesehen ist, um eine Bestimmung des neurophysiologischen grundlegenden Parameters des Patienten (2) durch die Parametererfassungseinheit (7) zu steuern, und um die Stimulation des Patienten (2) mit einem elektrischen Stimulationssignal durch die Stimulationseinheit (6) zu steuern,
wobei die Bestimmung des neurophysiologischen grundlegenden Parameters und die Stimulation durch die Steuereinheit (9) gesteuert werden, um in einer abwechselnden Weise zu erfolgen.

**10.** Vorrichtung nach den Ansprüchen 2 bis 9,
wobei die elektrischen Stimulationssignalsequenzen des elektrischen Stimulationssignals verschiedene Signalformen umfassen.

**11.** Vorrichtung nach Anspruch 10,
wobei die periodischen elektrischen Stimulationssignalsequenzen Impulse umfassen oder eine sinusförmige Signalwellenform haben.

**12.** Vorrichtung nach Anspruch 11,
wobei die Impulslänge eines Impulses in einer elektrischen Stimulationssignalsequenz mit einer Erhöhung der Stimulationsfrequenz der elektrischen Stimulationssignalsequenz gesenkt wird.

**13.** Vorrichtung nach den Ansprüchen 9 bis 12,

wobei die am Patienten (2) angebrachten Elektroden (3) zwischen dem Anlegen eines elektrischen Stimulationssignals an den Patienten (2) in einer ersten Betriebsart und dem Ableiten eines von dem Patienten (2) aufzuzeichnenden Signals in einer zweiten Betriebsart schaltbar sind,
wobei das Schalten der Elektroden (3) durch die Steuereinheit (9) gesteuert ist.

**14.** Vorrichtung nach Anspruch 13,
wobei ein Speicher vorgesehen ist, um die mittels der angebrachten Elektroden (3) in der zweiten Betriebsart aufgezeichneten Signale zu speichern.

## Revendications

**1.** Appareil (1) destiné à stimuler un patient (2), comprenant :

une unité de stimulation (6) destinée à appliquer un signal de stimulation électrique ayant au moins un paramètre de signal de stimulation variable, sachant que l'on fait varier en permanence ledit paramètre de signal de stimulation dans une plage de paramètre calculée autour d'un paramètre fondamental neurophysiologique déterminé dudit patient (2),
sachant qu'une unité de détermination de paramètre (7) est prévue pour déterminer au moins un paramètre fondamental neurophysiologique, sachant que ladite unité de détermination de paramètre (7) comprend un moyen de détecter un signal électrique provenant du cerveau (5) dudit patient (2) et un moyen d'analyser ledit signal électrique détecté pour déterminer ledit paramètre fondamental neurophysiologique dudit patient (2),
sachant que ledit paramètre fondamental neurophysiologique déterminé comprend une fréquence fondamentale neurophysiologique dudit patient (2), sachant que ladite unité de détermination de paramètre (7) comprend des électrodes électriques (3) configurées pour être attachées à la surface de la peau dudit patient (2), sachant que les électrodes (3) sont aptes à appliquer le signal de stimulation électrique et à détecter un signal électrique provenant du cerveau (5).

**2.** L'appareil selon la revendication 1,

sachant que ladite unité de stimulation (6) génère un signal de stimulation électrique composé de plusieurs séquences de signal de stimulation électrique périodiques ayant chacune une fréquence de stimulation correspondante,
sachant que lesdites séquences de signal de stimulation électrique sont séparées par des interruptions de signal d'une période prédéterminée et ont des fréquences de stimulation différentes au sein de ladite plage de fréquence calculée.

3. L'appareil selon les revendications 1 à 2,
sachant que ladite unité de détermination de paramètre (7) comprend

- un moyen d'enregistrement destinés à enregistrer un signal EEG dudit patient (2) et
- un moyen de transformation destinés à transformer le signal EEG enregistré en ladite fréquence fondamentale neurophysiologique dudit patient (2).

4. L'appareil selon la revendication 3,
sachant que lesdits moyens de transformation sont formés par des moyens de transformation de fréquence de Fourier aptes à effectuer une transformation de fréquence de Fourier dudit signal EEG transformé ou par des moyens d'auto-régression aptes à effectuer une auto-régression dudit signal EEG enregistré.

5. L'appareil selon la revendication 1,
sachant que ladite unité de détermination de paramètre (7) est apte à détecter une fréquence de fusion de papillotement en tant que la fréquence fondamentale neurophysiologique dudit patient (2).

6. L'appareil selon les revendications 3 à 5,
sachant que
les électrodes (3) sont aptes à appliquer un signal de stimulation électrique audit patient (2) et à enregistrer un signal EEG dudit patient (2).

7. L'appareil selon la revendication 5,

sachant que ladite unité de stimulation (6) stimule ledit patient (2) avec le signal de stimulation électrique ayant une fréquence de stimulation réglable qui est augmentée pour provoquer une légère sensation de lumière scintillant rythmique dudit patient (2) jusqu'à ce que ladite fréquence de stimulation provoque une sensation de lumière constante du patient (2),
sachant que la fréquence de stimulation réglée est la fréquence de fusion de du lumière scintillante formant ladite fréquence fondamentale neurophysiologique dudit patient (2).

8. L'appareil selon les revendications 1 à 7,
sachant que ladite unité de stimulation (6) fait varier de manière continue ou discontinue la fréquence de stimulation dudit signal de stimulation électrique.

9. L'appareil selon les revendications 1 à 8,

sachant qu'une unité de commande (9) est prévue pour commander une détermination dudit paramètre fondamental neurophysiologique dudit patient (2) par l'unité de détection de paramètre (7) et pour commander la stimulation du patient (2) avec un signal de stimulation électrique par ladite unité de stimulation (6),
sachant que la détermination dudit paramètre fondamental neurophysiologique et la stimulation sont commandées par ladite unité de commande (9) de manière à être effectuées en alternance.

10. L'appareil selon les revendications 2 à 9,
sachant que les séquences de signal de stimulation électrique dudit signal de stimulation électrique comprennent différentes formes de signal.

11. L'appareil selon la revendication 10,
sachant que lesdites séquences de signal de stimulation électrique périodiques comprennent des impulsions ou ont une forme d'onde de signal sinusoïdale.

12. L'appareil selon la revendication 11,
sachant que la longueur d'impulsion d'une impulsion au sein d'une séquence de signal de stimulation électrique est diminuée avec l'augmentation de la fréquence de stimulation de ladite séquence de signal de stimulation électrique.

13. L'appareil selon les revendications 9 à 12,

sachant que lesdites électrodes (3) attachées audit patient (2) sont commutables entre l'application d'un signal de stimulation électrique audit patient (2) dans un premier mode de fonctionnement et le fait de dériver un signal devant être enregistré depuis ledit patient (2) dans un deuxième mode de fonctionnement, sachant que la commutation desdites électrodes (3) est commandée par ladite unité de commande (9).

14. L'appareil selon la revendication 13, sachant qu'une mémoire est prévue pour stocker les signaux enregistrés au moyen desdites électrodes (3) attachées dans ledit deuxième mode de fonctionnement.

Fig 1

Fig 2

Fig 3

EP 3 326 688 B1

Fig 4

Fig 5

EP 3 326 688 B1

Fig 6

Fig 7A

Fig 7B

EP 3 326 688 B1

Fig 8

Start

determining
flicker fusion
frequency

~ S7

calculating
frequency
range

~ S8

selecting
modulation
of
stimulation
signal

↓ S9

application
of
stimulation
signal

~ S10

Stop

Fig 5

79 Hz:

80 Hz:

81 Hz:

Fig 10A

Fig 10B

Fig 10C

Fig 11

Fig 12

Fig 13

Fig 14

**EP 3 326 688 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1145735 A2